# EUROPÄISCHE PATENTSCHRIFT

(11) **EP 1 068 233 B1**
(45) Veröffentlichungstag und Bekanntmachung des Hinweises auf die Patenterteilung: **07.06.2006**
(21) Anmeldenummer: 99910361.7
(22) Anmeldetag: 18.03.1999
(51) Int. Cl.: C07K 14/47, A61K 38/17, G01N 33/68

(54) **TRANSKRIPTIONSFAKTOREN UND DEREN VERWENDUNG**
TRANSCRIPTION FACTORS AND THEIR USE
FACTEURS DE TRANSCRIPTION ET LEUR UTILISATION

(30) Priorität: 06.04.1998 DE 19815331
(43) Veröffentlichungstag der Anmeldung: 17.01.2001
(73) Patentinhaber: Forschungszentrum Karlsruhe GmbH, 76133 Karlsruhe (DE); Universiteit Leiden, 2300 RA Leiden (NL)
(72) Erfinder: ANGEL, Peter, D-76344 Eggenstein-Leopoldshafen (DE); HERRLICH, Peter, D-76229 Karlsruhe (DE); VAN DAM, Hans, NL-2324 WR Leiden (NL); VAN DER ERB, Alex, NL-2341 TW Oegstgeest (NL)
(74) Vertreter: Rückert, Friedrich
(86) Internationale Anmeldenummer: PCT/EP1999/001805
(87) Internationale Veröffentlichungsnummer: WO 1999/051635

(56) Entgegenhaltungen:
- WO-A-97/12988
- US-A- 5 721 340
- BAXEVANIS A D ET AL: "INTERACTIONS OF COILED COILS IN TRANSCRIPTION FACTORS: WHERE IS THE SPECIFICITY?" CURRENT OPINION IN GENETICS & DEVELOPMENT, Bd. 3, 1. Januar 1993, Seiten 278-285, XP002022679
- M. SCHUERMANN ET AL.: "Non-leucine residues in the leucine repeats of Fos and Jun contribute to the stability and determine the specificity of dimerization" NUCLEIC ACID RESEARCH, Bd. 19, Nr. 4, 1991, Seiten 739-746, XP002106708
- SCHMIDT-DOERR T ET AL: "CONSTRUCTION, PURIFICATION, AND CHARACTERIZATION OF A HYBRID PROTEIN COMPRISING THE DNA BINDING DOMAIN OF THE LEXA REPRESSOR AND THE JUN LEUCINE ZUPPER: A CIRCULAR DICHROISM AND MUTAGENESIS STUDY" BIOCHEMISTRY, Bd. 30, Nr. 40, 8. Oktober 1991, Seiten 9657-9664, XP000647702
- O.D. MONERA ET AL.: "Formation of parallel and antiparallel coiled -coils controlled by the relative positions of alanine residues in the hydrophobic core" J. BIOL. CHEM., Bd. 271, Nr. 8, Februar 1996, Seiten 3995-4001, XP002106709
- P.FERNANDEZ-SILVA ET AL.: "The human mithocondrial transcription termination factor is a multizipper protein but binds to DNA as a monomer..." EMBO J., Bd. 16, Nr. 5, 1997, Seiten 1066-1079, XP002106710

## Beschreibung

Die Erfindung betrifft Transkriptionsfaktoren mit einer DNA-Bindungsdomäne, bestehend aus einer basischen Domäne und einer benachbarten Leuzin-Reißverschluß Domäne sowie deren Verwendung.

Van Straaten et al. und Angel et al. haben solche Transkriptionsfaktoren beschrieben (van Straaten, F., Müller, R., Curran, T., Van Beveren, C.and Verma, I. M. (1983) Proc. Natl. Acad. Sci. U.S.A. 80, 3183-3187; Angel, P., Allegretto, E.A., Okino, S.T., Hattori, K., Boyle, W.J., Hunter, T., and Karin, M. (1988) Nature 332, 166-171). Vinson et al. beschreiben den Mechanismus der Dimerisierung dieser Transkriptionsfaktoren mit Hilfe der Reißverschlußdomänen (Vinson, C. R., Hai, T., and Boyd, S. M. (1993). Genes Dev. 7, 1047-1058.). Dabei wurde postuliert, dass in der Reißverschlußdomäne die Aminosäure in g Position (drei Positionen carboxyterminal von Leucin) des einen Dimerenpartners mit der Aminosäure in der nächsten e Position (fünf Positionen carboxyterminal) des anderen Dimerenpartners interagiert, wobei durch diese Interaktionen die Spezifizität bestimmt wird. Diese Bindungsmechanismen, die an isolierten Peptiden etabliert wurden, die nur die Reißverschlußdomäne umfassen, lassen sich jedoch nicht allgemein auf das Bindungsverhalten der vollständigen Proteine an DNA-Sequenzen übertragen.

A. D. Baxevanis und C. R. Vinson, Current Opinions in Genetics & Development, Bd. 3, 1. Januar 1993, S. 278-285, beschreiben die Transkriptionsfaktoren fos, Max und USF, die in den Positionen ei und gi geladene Aminosäuren aufweisen. T. Schmidt-Doerr, P. Oertel-Buchheit, C. Pernelle, L. Bracco, M. Schnarr und M. Granger-Schnarr, Biochemistry 1991, Bd. 30, 8. Oktober 1991, S. 9657-9664, stellen die Aminosäuresequenzen fos1, fos2, jun1 und jun2 dar und zeigen, dass Mutagenisierung der Positionen e4, g4 und e5 die Dimerisierungskapazität des Leuzin-Zippers beeinflusst.

M. Schuermann, J. B. Hunter, G. Hennig und R. Müller, Nucleic Acid Research, Bd. 19, 1991, S. 739-746, offenbaren, dass Aminosäuren in den Positionen a, e und g für die Spezifität der Interaktion von Leuzin-Zipper Proteinen wesentlich sind und beschreiben fos und jun Proteine mit Mutationen in der Leuzin-Zipper Domäne. Aus der US-A-5 721 340 ist eine Variante des GCN4 Leuzin-Zippers als Tetramerisierungsdomäne bekannt, die Lys in Position e1 und Glu in Position e2 aufweist.

Aufgabe der Erfindung ist es, modifizierte Transkriptionsfaktoren zur Verfügung zu stellen, deren Bindungsverhalten zueinander und zu DNA-Sequenzen manipulierbar ist.

Gelöst wird diese Aufgabe durch die Merkmale des Patentanspruchs 1. Anspruch 2 beschreibt eine vorteilhafte Ausgestaltung der Erfindung. Die Ansprüche 3, 4, und 5 nennen besondere Verwendungen der Transkriptionsfaktoren.

Ein besonderer Vorteil der erfindungsgemäßen Transkriptionsfaktoren besteht darin, dass es möglich wird, durch geeignete Substitution von Aminosäuren ganz gezielt Reaktionspartner auszuwählen, und zwar nicht mit artifiziellen Peptiden, sondern im Kontext des authentischen Proteins. Vor allem ist es möglich, durch geeignete Substitution das Bindungsverhalten an DNA-Sequenzen zu verändern; eine Aussage die mit den bisher verwendeten synthetischen Peptiden nicht möglich war.

Die Erfindung wird im Folgenden anhand von Ausführungsbeispielen mit Hilfe der Tabelle näher erläutert.

Dabei zeigt die Tabelle die Proteinsequenz im Bereich des Leuzinreißverschlusses für verschiedene Substituenten bei zwei unterschiedlichen Transkriptionsfaktoren cJun (Angel et al., 1987, siehe oben) und ATF2 (Maekawa, T., Sakura, H., Kanei-Ishii, C., Sudo, T., Yoshimura, T., Fujisawa, J.-i., Yoshida, M., and Ishii, S. (1989) EMBO J. 8, 20-23-2028.).

Die Tabelle zeigt zeilenweise die Aminosäurefolge (Position 280 - 310) für 14 Substituenten von cJun und drei von ATF2 (Position 380 - 410). e1 bis e5 und g1 -g5 sind die möglichen Substitutionspositionen. Die Buchstaben entsprechen dem üblichen Aminosäuren-Code.

### Ausführliche Substituentenbeschreibung:

### cJun-m0:

1) hat ohne DNA im Vergleich mit Wildtyp cJun erhöhte Affinität für den cJun Dimeren -Partner cFos (van Straaten et al., 1983, siehe oben)
2) bindet gut an DNA wenn es mit cFos dimerisiert, aber schlecht wenn es mit ATF2 angeboten wird, da Jun-m0 nicht oder nur geringfügig an ATF2 dimerisiert, bindet sehr schlecht als Jun-m0 /Jun-m0 Homodimer.
3) aktiviert im Vergleich mit Wildtyp cJun in mehrere Zelltypen das Jun/Fos-abhängige Reporter- Gen 5xcollTREtata-luciferase relativ effizient, das Jun/ATF2--abhängige Reporter- Gen 5xjun2-tata-Luciferase nur sehr ineffizient
4) bindet nicht an DNA mit ATF2-m5 (sie unten) bindet nicht an DNA mit wt cJun oder JunD (Hirai, S.-I., Ryseck, R.-P., Mechta, F., Bravo, R., and Yaniv, M. (1989). EMBO J. 8, 1433-1439.)

### cJun-m35:

1) bindet ohne DNA im Vergleich mit wt cJun etwas besser an ATF2 als an cFos
2) bindet im Vergleich mit wt Jun viel stärker an DNA als Jun-m35/Jun-m35 Homodimere oder wtJun/Jun-m35 Heterodimere, bindet auch gut an DNA als Jun/ATF2, bindet relativ schwach an DNA als Jun/Fos;
3) aktiviert im Vergleich mit wt cJun den oben schon genannten Jun/Fos-abhängigen Reporter schlechter und den Jun/ATF2-abhängige Promoter besser.
4) bindet sehr gut an DNA mit ATF2-m5 (siehe unten)
5) bindet relativ gut an DNA mit JunD

### cJun-m2:

1) bindet ohne DNA relativ schlecht an ATF2, gut an cFos,
2) bindet ohne DNA nicht oder sehr schlecht an JunD
3) bindet schlecht an DNA mit wt cJun und mit JunD
4) bindet schlecht an DNA mit ATF2-m5 (siehe unten)

### cJun-ml:

1) bindet ohne DNA im Vergleich mit wt Jun viel schwächer an cFos, aber gut an ATF2
2) bindet gut an DNA wenn dimerisiert mit ATF2, aber schlecht wenn zusammen mit cFos angeboten, da mit cFos schlecht dimerisierend,
3) aktiviert im Vergleich mit Wildtyp cJun in mehreren Zelltypen das Jun/Fos-abhängige Reportergen 5xcollTRE-tataluciferase sehr uneffizient, das Jun/ATF2-abhängige Reportergen 5xjun2-tata-luciferase aber sehr gut
4) bindet sehr gut an DNA mit ATF2-m5 (siehe unten)
5) bindet ohne DNA relativ stark an wt cJun und JunD

### cJun-m0,3:

1) bindet schlecht an DNA mit ATF2 und als Homodimer
2) bindet schlecht an DNA mit ATF2-m5 (siehe unten)

### cJun-m0,35:

1) unterscheidet sich von cJun-m35 (siehe oben) durch schwächere Bindung an DNA als Homodimer
2) relativ schwache Bindung an DNA mit wt-cJun und JunD
3) unterscheidet sich von cJun-m35 in schwächere Bindung an DNA mit ATF2-m5

### cJun-m0,2:

bindet sehr schlecht an DNA als Homodimer, relativ schwach mit ATF2, aber gut als Dimer mit cFos

### cJun-m0,1:

1) bindet ohne DNA im Vergleich mit wt-Jun schwächer an cFos, aber gut an ATF2
2) bindet gut an DNA wenn dimerisiert mit ATF2, aber relativ schlecht wenn zusammen mit cFos angeboten, da mit cFos schlecht dimerisierend
3) aktiviert im Vergleich mit Wildtyp cJun in mehreren Zelltypen das Jun/Fos-abhängige Reportergen 5xcollTRE-tata-Luciferase unefficient, das Jun/ATF2-abhängige Reportergen 5xjun2-tata-luciferase aber gut
4) bindet im Vergleich mit m1 (siehe oben) relativ schwach an wt-cJun und JunD
5) bindet schwach an DNA mit ATF2-m5

### cJun-m5:

1) bindet ohne DNA relativ schlecht an ATF2, aber gut an cFos
2) bindet relative stark an DNA mit Fos und als m5/m5 Homodimer
3) bindet ohne DNA sehr schwach an JunD

### cJun-m0,5:

1) bindet sehr schwach an DNA als Homodimer
2) bindet sehr schlecht an DNA mit ATF2-m5

### cJun-m3,5:

1) bindet sehr stark an DNA als Homodimer
2) bindet sehr schlecht an DNA wenn dimerisiert mit ATF2-m5 (sie unten)

### cJun-m35,5:

1) bindet sehr stark an DNA als Homodimer,
2) bindet relativ stark an DNA mit ATF2-m5 (siehe unten)

### cJun-m2,5:

1) bindet ohne DNA sehr schwach an ATF2, aber bindet noch an cFos
2) bindet ohne DNA schwach an JunD

### cJun-m1,5:

1) bindet ohne DNA sehr schlecht an cFos, relativ gut an ATF2
2) bindet nicht oder sehr schlecht an DNA als Jun/Fos, nur schwach als Jun/ATF2
3) aktiviert im Vergleich mit wt-cJun den oben erwähnte Jun/Fos-abhängigen Reporter schlechter und den Jun/ATF2-abhängigen Promoter besser.
4) bindet ohne DNA sehr stark an JunD
5) bindet schwach an DNA mit ATF2-m5 (siehe unten)

### ATF2-m1:

1) bindet sehr schwach an DNA als Homodimer und als Heterodimere mit cJun-wt
2) bindet gut an DNA mit cJun-m35 (siehe oben)

### ATF2-m2:

1) bindet schwach an DNA als Homodimer, aber im Gegensatz mit ATF2-m1 und m5 (siehe unten) noch relativ gut mit wt-cJun
2) bindet gut an DNA mit cJun-m35 (siehe oben)

### ATF2-m5:

1) bindet ohne DNA im Vergleich mit wt-ATF2 relativ schwach an wt-cJun
2) bindet nicht an DNA als Homodimer oder als Heterodimer mit wt-cJun
3) bindet gut an DNA mit cJun-m35 (siehe oben)
4) bindet schwach an DNA als ATF2/cFos (wt-ATF2 bindet nicht an DNA mit cFos

Angewendete Methoden bei der Charakterisierung der Mutanten:
a) Protein-interaktion ohne DNA wurde gemessen mit einer so genannten "mammalian two-hybrid Analyse" für welche der Mutante-Reißverschluß fusioniert wurde mit der DNA-Bindungsdomäne des Hefe-Proteins GAL4 (GAL4-D). Die Interaktion mit cFos, ATF2 oder JunD wurde quantifiziert durch Einbringen eines dieser Proteine mit dem cJun-GAL4-D Fusionsprotein in die gleiche Zelle, zusammen mit einem GAL4-abhangigen Reportergen. Die zu quantifizierende Aktivierung des Reporter- Konstrukts ist ein Maß für die Protein- Protein Interaktion.
b) DNA Bindung als Jun/Jun und Jun/Fos Dimere wurde gemessen an einem 20 Basenpaare langen doppelsträngigen Oligonukleotid mit als essentieller DNA Sequenz dem Kernmotiv TGAGTCA, für DNA Bindung als Jun/ATF2 oder ATF2/ATF2 Dimere wurde ein ähnliches Oligonukleotid mit Kernmotiv TTACCTCA benutzt.

### Beispiele für Deaktivierung und Hyperaktivierung von Transkriptionsfaktoren

1. Deaktivierung durch Deletion oder Mutation der kompletten, oder bestimmter Teile der Transaktivierungsdomäne:
   a) Deletion der gesamten Transaktivierungsdomäne von cJun (Aminosäure 6 bis 194) oder ATF2 (Aminosäure 1 - 110 von ATF2).
   b) Deletion einzelner Subdomänen der Transaktivierungsdomäne von cJun (Region I, II, und/oder III; HOB Domäne; Delta Domäne als Andockstelle für MAP Kinasen)
   c) Mutation der Phosphorylierungsplätze Ser63 und/oder Ser73, Thr89, Thr91, Thr93 von cJun oder Thr69, Thr71 und Ser90 von ATF2: Substitution durch einer Ala oder Leu Aminosaure
   d) Deletion der Zinc-finger Domäne im aminoterminalen Teil von ATF2; Deletion der Bindungsdomäne in ATF2 für Coactivatoren.
2. Deaktivierung durch Deletion oder Mutation von Teilen der DNA-Bindungsdomäne (z. B. basische Domäne: Blockierung der DNA-Bindung, nur noch Dimerisierung möglich).
3. Hyperaktivierung durch Änderungen an Transaktivierungsdomänen:
   a) Substitution Ser63 und/oder Ser73, Thr89,91,93 von cJun und Thr69 und/oder Thr71, Ser90 von ATF2 durch Asp.
   b) Fusion von cJun oder ATF2 mit der hyperaktiven Transaktivierungsdomäne des Herpes Simplex Virus VP16 Proteins.

### Beispiele für Anwendung als Therapeutika:

a) Das Verhältnis von cJun-Fos Aktivität und cJun-ATF2 Aktivität spielt eine wichtige Rolle bei programmiertem oder anderem Zelltod. Bringt man nun eine oder ein Gemisch der folgenden cJun- Mutanten m1, m0,1, m35 oder m1,5 in normaler, oder besser, in hyperaktiver Form(siehe oben) in eine Zelle, dann wird nur die cJun -ATF2 Aktivität hoch reguliert. Kombinationen von normalem oder hyperaktivem ATF2-m5 oder ATF2-ml zusammen mit normalen und hyperaktiven cJun-m1, m0,1, m35, oder m1,5 Mutanten wirken in bestimmten Zelltypen noch effizienter. Damit sind diese Mutanten optimal geeignet um Zelltod auszulösen. Diese Mutanten sind daher geeignete Therapeutika zur Behandlung von Krebsarten bei denen der Zelltod durch Aktivierung von cJun-ATF2 Aktivität eine Rolle spielt (z. B. Krebszellen des Ovars, die empfindlich gegen Cisplatin sind).
b) Wie oben erwähnt hat die Einführung von cJun-m0 in bestimmte Zelltypen das Resultat, dass nur Jun-Fos Aktivität effizient induziert wird. In Zellen von Patienten in welchen unerwünschter (programmierter) Zelltod stattfindet, kann eine normale oder besser hyperaktive Version von cJun mO also die Bilanz von Jun/Fos Aktivität im Vergleich mit Jun/ATF2 Aktivität beeinflussen und so den unerwünschten Zelltod verhindern. Diese Versionen von m0 sind also geeignete Therapeutika solche Fälle von unerwünschtem Zelltod zu hemmen.
c) Metastasierung von Krebszellen wird in bestimmten Fälle induziert oder verstärkt durch Aktivierung von Jun/Fos-Aktivität z. B. durch Induktion von Genen, die für Zelloberflächen-Proteine kodieren: z. B. das CD44 Protein. Jun/fos beeinflusst dadurch sowohl die Wanderfähigkeit als auch die Einnistung der Krebszellen in anderen Geweben und Organen. Hemmung von Metastasierung durch spezifische Hemmung von Jun/Fos Aktivität, durch das Einbringen einer deaktivierten Version von cJun-m0, z.B. einer m0 Mutanten in welcher die Aminosäuren 6-194 deletiert sind, in die Zellen.
d) Die cJun, ATF2 und cFos Proteine spielen eine sehr wichtige Rolle in bestimmten Programmen der Embryonalentwicklung und anderer Arten von Zelldifferenzierung. Normale, deaktivierte oder hyperaktiven Formen der Mutanten cJun-m0, m1, m1,5 und ATF2-m1 und -m2 können durch spezifische Hemmung oder Aktivierung von Jun/Fos, Jun/ATF2 und ATF2/ATF2 Aktivität die Zelldifferenzierung beeinflussen und dadurch Fehler in der Embryonalentwicklung korrigieren. Ebenfalls können die Mutanten bestimmte Arten von Krebszellen zu Zelldifferenzierung anregen und so ihr Wachstum hemmen.

Vorgeschlagene Methode um die mutanten Transkriptionsfaktoren in vivo einzubringen:
a) als Gen Konstrukt via Gen-Therapie: z. B. in einem Adenovirusvektor oder in einem retroviralen Vector (Verma, I.M. und Somia, N. (1997) Nature 389; 239-242; Mulligan, R.C. (1993) Science 260, 926-932
b) als kleines Peptid analog den Mdm2/p53 Peptiden.

### Beispiele von Testsystemen für Medikamente:

Wie oben beschrieben, unterscheiden normale, hyperaktive oder (teilweise) deaktivierte Formen von cJun-mO, oder m1, m0,1, m35 sich in ihre Kapazität Jun/Fos, Jun/Jun und Jun/ATF2 Dimere zu bilden, und Jun/Fos oder Jun/ATF2 abhängige Reporter Gene anzuschalten. Das bietet die Möglichkeit in biologischen Prozessen, die von mehreren verschiedenen Jun-Dimere reguliert werden (wie bestimmte Embryonalentwicklung, Krebsarten, programmierte oder andere Arte von Zelltod, Überlebensfunktion von Zellen, verstärkte Stress-Responsen und Zell-Differenzierung), die Rolle von der unterschiedlichen Jun-Dimeren zu untersuchen.

Zusätzlich bieten diese Mutanten die Möglichkeit entsprechende Medikamente auf ihre Spezifität auf die verschiedene Arten von Jun-Dimeren und die dazugehörenden biologischen Prozesse auszutesten.

Substanzen diverser Klassen können auf spezifische Interferenz mit einem bestimmten Dimerenpaar getestet werden.

So kann man Medikamente austesten die bestimmte Aspekte vom Krebswachstum, z. B. Metastasierung, spezifisch hemmen. Auch kann man Medikamente austesten die von Jun/ATF2 induzierten Zelltod hemmen oder verstärken sollen.

Weiter bietet es die Möglichkeit, mit Medikamenten spezifisch in durch Jun/Fos, Jun/ATF2 oder ATF2/ATF2 kontrollierte Zelldifferenzierung einzugreifen, zum Beispiel um Fehler während der Embryonalentwicklung zu korrigieren und/oder zu blockieren.

Vorgeschlagene Testsysteme zum Austesten von Medikamenten: *In vitro* Zellkulturen und transgene Mäuse, in welchen die mutanten Transkriptionfaktoren durch Standard-Gentechnologie exprimiert werden.

### Allgemeine Anwendung von mutanten Transkriptionsfaktoren als Medikamente oder zum Austesten von Medikamenten:

Die Spezifitätsmechanismen sind auf andere Proteine, die über bZIP (basische Domäne-Leuzin-Reißverschluss) verfügen und auf Proteine die über einen Leuzin-Reißverschluss interagieren, übertragbar. (Vorbilder sind JunB, JunD, ATFa, ATF3, CREB, ATF1, MAF, CEBP (Hurst, H.C. 1994, Transcription factors: 1: bZip proteins. Protein profile 1, 123-168). Deshalb kann man die normalen Reißverschlüsse von diesen bZIP Proteinen ersetzen durch einen der oben genannten mutanten Reißverschlüsse und so, wie oben beschrieben für Jun/Fos und Jun/ATF2, die Rolle in biologischen Prozesse von einzelnen Dimeren gebildet durch zum Beispiel JunD oder ATF3 untersuchen. Deaktivierte und hyperaktive Versionen von diesen anderen bZIP Transkriptionsfaktoren können so benutzt werden, um therapeutisch in mehrere Arten von Krankheiten einzugreifen, in welchen bZIP Transkriptionsfaktoren eine Rolle spielen, wie zum Beispiel Autoimmun -Krankheiten, Krebs, genetischen Abweichungen und Embryonalentwicklungsprobleme.

Sowie schon oben beschrieben können mit diesen Mutanten ebenfalls Medikamente ausgetestet werden die spezifisch für die einzelnen ATF3, ATFa, JunD, CEBP etc. Dimeren sind. Viele Arten von Krankheiten, bei denen die bZIP Proteine eine Rolle spielen, können so beeinflusst werden.

### Allgemeine Anwendung von den Leuzin-Reisverschlussdomäne-Mutanten für spezifisches eingreifen in Protein-Protein Interaktion in vitro und in vivo

Eine große Menge von Proteinen interagiert mit anderen Proteinen über ihre Leuzin-Reißverschlussdomäne. Deshalb können die oben genannten mutanten Reißverschlussdomänen mit veränderter Interaktionsspezifität benutzt werden, um selektiv in Interaktionen von allen einen Leuzin-Reißverschluss enthaltenden Proteinen einzugreifen. Die normale Reißverschlussdomäne wird ersetzt durch einen oben beschriebenen geeigneten mutanten Reißverschluss, wie zum Beispiel Jun-m0, ATF2-m5 oder cJun-m1,5.

Des Weiteren können durch das Eliminieren mit Gentechnologie von anderen Typen von Proteininteraktionsdomänen und das Einbringen von oben beschrieben mutanten Reißverschlüssen, Proteine sehr kontrolliert aneinander gekoppelt werden.

### Mutante Reißverschlüsse als Kopplungspartner von verschiedenen Wirkungsgruppen.

Die Reißverschlussdomäne von cJun m0 hat bei neutralem pH eine starke positive Ladung, weil cJun-m1, und cJun-m1,5 eine sehr starke negative Ladung tragen. Deshalb sind m0 einerseits und m1 anderseits sehr geeignet als Kopplungspartner von Biomolekülen.

Mit Hilfe der üblichen genetischen und (bio)chemischen Methoden können die m0, m1 und m1,5 Polypeptide an andere Wirkungskomponenten gekoppelt werden. Diese mutanten Domänen sind zum Beispiel sehr geeignet für die Anwendung im Drogen-Targeting bei bestimmten Zelltypen. Man kann bestimmte biologisch aktive Proteine via Gentechnologie mit dem m0 Reißverschluss fusionieren und mit Gentherapie in den Körper hineinbringen. Die zu targeten Zellkomponente wird gleichfalls mit dem m1,5 Reisverschluss markiert.

Modellierung von nuklearen Rezeptoren.

Neben der eigentlichen Rolle als Transkriptionsfaktoren wirken die Jun- Dimeren auch in der Wechselwirkung mit anderen Transkriptionsfaktoren. In vielen Fällen resultiert diese Wechselwirkung in gegenseitiger Hemmung, z. B. hemmt der Glucocorticoid- Rezeptor die Aktivität von Jun.: Fos an Jun: Fos Zielgenen. Umgekehrt interferiert Jun: Fos mit der Gen aktivierenden Wirkung des Glucocorticoid-Rezeptors. Diese zweite Art der Transkriptionsfaktorwirkung ist die Basis der Entzündungshemmung durch Cortisol, der Krebs hemmenden Wirkung der Retinoide und sie sind lebenswichtig für den Organismus. Die komplexen Wechselwirkungen der erfindungsgemäßen Mutanten und Peptide mit nuklearen Rezeptoren können benutzt werden, diese Wechselwirkung mit den oben genannten Methoden zu beeinflussen. Außerdem können z. B. die Jun- Mutanten zur Einrichtung eines Screeningtests für entzündungshemmende und antitumorgene Liganden der nuklearen Rezeptoren eingesetzt werden.

Bei allen Arten von Krebs sind die von Jun und seinen Partnern ausgelösten Eigenschaften involviert. Jun nimmt dabei eine Zentralposition ein. Die neuen Jun-Mutanten ermöglichen spezielle Testsätze zum Testen von interferierenden Drogen. Außerdem kann mit den oben genannten Methoden des Peptid- oder Gen einbringens das krebsartige Wachstum beeinflusst werden.

Jun: Fos und Jun: Atf2 induzieren komplementäre Programme in der krebsartigen Zellentartung: Wachstumsfaktor-Unabhängigkeit bzw. Wachstum in semisolidem Medium.

Mit den oben beschriebenen Mutanten können Krebseigenschaften anderen Ursprungs, z. B. Adenoviren verursachte Transformationen beeinflusst und damit Therapeutika auf ihre spezifische Wirksamkeit getestet werden.

**Tabelle:**

| | *e1* | *g1* | *e2* | *g2* | *e3* | *g3* | *e4* | *g4* | *e5* | |
|---|---|---|---|---|---|---|---|---|---|---|
| cJun wt | | | | | | | | | | Sequenz 2 |
| m0 | K | K | K | Q | A | T | R | Q | K | Sequenz 3 |
| m35 | E | E | E | Q | A | T | R | Q | K | Sequenz 4 |
| m2 | E | K | K | Q | A | T | E | E | K | Sequenz 5 |
| m1 | E | E | E | Q | A | T | E | E | K | Sequenz 6 |
| m0,3 | K | K | E | Q | A | T | R | Q | K | Sequenz 7 |
| m0,35 | K | E | E | Q | A | T | R | Q | K | Sequenz 8 |
| m0,2 | K | K | K | Q | A | T | E | E | K | Sequenz 9 |
| m0,1 | K | E | E | Q | A | T | E | E | K | Sequenz 10 |
| m5 | E | K | K | Q | A | T | R | Q | E | Sequenz 11 |
| m0,5 | K | K | K | Q | A | T | R | Q | E | Sequenz 12 |
| m3,5 | E | K | E | Q | A | T | R | Q | E | Sequenz 13 |
| m35,5 | E | E | E | Q | A | T | R | Q | E | Sequenz 14 |
| m2,5 | E | K | K | Q | A | T | E | E | E | Sequenz 15 |
| m1,5 | E | E | E | Q | A | T | E | E | E | Sequenz 16 |

| | *e1* | *g1* | *e2* | *g2* | *e3* | *g3* | *e4* | *g4* | *e5* | |
|---|---|---|---|---|---|---|---|---|---|---|
| ATF2 wt | | | | | | | | | | Sequenz 17 |
| m1 | K | K | S | L | Q | R | R | E | K | Sequenz 18 |
| m2 | E | K | S | R | Q | V | R | E | K | Sequenz 19 |
| m5 | K | K | S | R | Q | V | R | E | K | Sequenz 20 |

## Patentansprüche

1. Transkriptionsfaktoren mit einer DNA-Bindungsdomäne, bestehend aus einer basischen Domäne und einer benachbarten Leuzin-Reißverschluss Domäne mit folgenden Aminosäuren:
L e1 x g1 x x x L e2 x g2 x x x L e3 x g3 x x x L e4 x g4 x x x L e5 x (Sequenz 1), wobei L Leuzin, e1,g1, e2, g2, e3, g3, e4, g4, und e5 mögliche Substitutionsstellen und x die übrigen Aminosäuren sind, **gekennzeichnet durch** einen der folgenden Sätze von Substituenten an den neuen Substitutionsstellen e1,g1, e2, g2, e3, g3, e4, g4, und e5:
| e1 | g1 | e2 | g2 | e3 | g3 | e4 | g4 | e5 | |
|---|---|---|---|---|---|---|---|---|---|
| K | K | K | Q | A | T | R | Q | K | Sequenz 3 |
| E | E | E | Q | A | T | R | Q | K | Sequenz 4 |
| E | K | K | Q | A | T | E | E | K | Sequenz 5 |
| E | E | E | Q | A | T | E | E | K | Sequenz 6 |
| K | K | E | Q | A | T | R | Q | K | Sequenz 7 |
| K | E | E | Q | A | T | R | Q | K | Sequenz 8 |
| K | K | K | Q | A | T | E | E | K | Sequenz 9 |
| K | E | E | Q | A | T | E | E | K | Sequenz 10 |
| E | K | K | Q | A | T | R | Q | E | Sequenz 11 |
| K | K | K | Q | A | T | R | Q | E | Sequenz 12 |
| E | K | E | Q | A | T | R | Q | E | Sequenz 13 |
| E | E | E | Q | A | T | R | Q | E | Sequenz 14 |
| E | K | K | Q | A | T | E | E | E | Sequenz 15 |
| E | E | E | Q | A | T | E | E | E | Sequenz 16 |
| K | K | S | L | Q | E | R | E | K | Sequenz 18 |
| E | K | S | R | Q | V | R | E | K | Sequenz 19 |
| K | K | S | R | Q | V | R | E | K. | Sequenz 20 |

2. Transkriptionsfaktor nach Anspruch 1, **dadurch gekennzeichnet, dass** dieser **durch die gesamte Deletion der Transaktivierungsdomäne oder die Deletion einzelner Subdomänen der Transaktivierungsdomäne von cJun oder durch bestimmte Mutation von Phosphorylierungsplätzen der Aminosäuresequenz 6 bis 194 bei cJun oder in der Aminosäuresequenz 1-110 bei ATF2** oder durch Deletion der Zinc-finger Domäne im aminoterminalen Teil von ATF2 oder der Deletion der Bindungsdomäne in ATF2 für Coactivatoren, oder durch Deletion oder Mutation von Teilen der DNA-Bindungsdomäne deaktiviert ist.

3. Transkriptionsfaktor nach Anspruch 1, **dadurch gekennzeichnet, dass** dieser durch Substitution von Ser63 und/oder Ser73, Thr89, Thr91, Thr93 von cJun oder Thr69 und/oder Thr71, Ser90 von ATF2, durch Asp, oder durch Fusion von cJun oder ATF2 mit der hyperaktiven Transaktivierungsdomäne des Herpes Simplex Virus VP16 Proteins, hyperaktiviert ist.

4. Transkriptionsfaktoren gemäß einem der Ansprüche 1, 2 oder 3 zur Verwendung als Arzneimittel.

5. Verwendung von Transkriptionsfaktoren gemäß einem der Ansprüche 1, 2 oder 3 zum Testen von Medikamenten in *in vitro* Zellkulturen und transgenenen Mäusen, in wellchen die mutanten Transkriptionsfaktoren exprimiert werden.

6. Verwendung der Reißverschlussdomänen der Transkriptionszaktoren gemäß einem der Ansprüche 1, 2 oder 3 zur Kopplung von verschiedenen Wirkungsgruppen, vorzugsweise von Biomolekülen.

## Claims

1. Transcription factors with a DNA binding domain, consisting of a basic domain and an adjacent leucine zipper domain with the following amino acids:
L e1 x g1 x x x L e2 x g2 x x x L e3 x g3 x x x L e4 x g4 x x x L e5 x (sequence 1), where L is leucine, e1, g1, e2, g2, e3, g3, e4, g4, and e5 are potential substitution sites, and x is the remaining amino acids, **characterized by** one of the following sequences of substituents at the new substitution sites e1, g1, e2, g2, e3, g3, e4, g4, and e5:
| e1 | g1 | e2 | g2 | e3 | g3 | e4 | g4 | e5 | |
|---|---|---|---|---|---|---|---|---|---|
| K | K | K | Q | A | T | R | Q | K | sequence 3 |
| E | E | E | Q | A | T | R | Q | K | sequence 4 |
| E | K | K | Q | A | T | E | E | K | sequence 5 |
| E | E | E | Q | A | T | E | E | K | sequence 6 |
| K | K | E | Q | A | T | R | Q | K | sequence 7 |
| K | E | E | Q | A | T | R | Q | K | sequence 8 |
| K | K | K | Q | A | T | E | E | K | sequence 9 |
| K | E | E | Q | A | T | E | E | K | sequence 10 |
| E | K | K | Q | A | T | R | Q | E | sequence 11 |
| K | K | K | Q | A | T | R | Q | E | sequence 12 |
| E | K | E | Q | A | T | R | Q | E | sequence 13 |
| E | E | E | Q | A | T | R | Q | E | sequence 14 |
| E | K | K | Q | A | T | E | E | E | sequence 15 |
| E | E | E | Q | A | T | E | E | E | sequence 16 |
| K | K | S | L | Q | E | R | E | K | sequence 18 |
| E | K | S | R | Q | V | R | E | K | sequence 19 |
| K | K | S | R | Q | V | R | E | K | sequence 20 |

2. Transcription factor according to Claim 1, **characterized by** this factor being deactivated **by the complete deletion of the transactivation domain or the deletion of individual subdomains of the transactivation domain of cJun or by the specific mutation of phosphorylation sites of the amino acid sequence 6 to 194 in cJun or of the amino acid sequence 1 -110 in ATF2** or by the deletion of the zinc finger domain in the amino-terminal part of ATF2 or by the deletion of the binding domain in ATF2 for coactivators, or by the deletion or mutation of parts of the DNA binding domain.

3. Transcription factor according to Claim 1, **characterized by** this transcription factor being hyperactivated by the substitution by Asp of Ser63 and/or Ser73, Thr89, Thr91, Thr93 of cJun or Thr69 and/or Thr71, Ser90 of ATF2 or by the fusion of cJun or ATF2 with the hyperactive transactivation domain of the herpes simplex virus VP16 protein.

4. Transcription factors according to one of Claims 1, 2, or 3 for use as pharmaceutical product.

5. Use of the transcription factors according to one of Claims 1, 2, or 3, for testing drugs in *in vitro* cell cultures and transgenic mice, in which the mutant transcription factors are expressed.

6. Use of the zipper domains of the transcription factors according to one of Claims 1, 2, or 3 for coupling various active groups, preferably biomolecules.

## Revendications

1. Facteurs de transcriptions ayant un domaine de fixation à l'ADN constitué d'un domaine basique et d'un domaine à fermeture éclair à leucine voisine aux acides aminés suivants :
L e1 x g1 x x x L e2 x g2 x x x L e3 x g3 x x x L e4 x g4 x x x L e5 x (séquence 1), L étant de la leucine, e1, g1, e2, g2, e3, g3, e4, g4 et e5 étant des endroits de substitution possibles et x les autres acides aminés, **caractérisés par** l'une des séquences de substituants suivants, aux nouveaux endroits de substitution e1, g1, e2, e3, g3, e4, g4, et e5 :
| E1 | g1 | e2 | g2 | e3 | g3 | e4 | g4 | e5 | |
|---|---|---|---|---|---|---|---|---|---|
| K | K | K | Q | A | T | R | Q | K | séquence 3 |
| E | E | E | Q | A | T | R | Q | K | séquence 4 |
| E | K | K | Q | A | T | E | E | K | séquence 5 |
| E | E | E | Q | A | T | E | E | K | séquence 6 |
| K | K | E | Q | A | T | R | Q | K | séquence 7 |
| K | E | E | Q | A | T | R | Q | K | séquence 8 |
| K | K | K | Q | A | T | E | E | K | séquence 9 |
| K | E | E | Q | A | T | E | E | K | séquence 10 |
| E | K | K | Q | A | T | R | Q | E | séquence 11 |
| K | K | K | Q | A | T | R | Q | E | séquence 12 |
| E | K | E | Q | A | T | R | Q | E | séquence 13 |
| E | E | E | Q | A | T | R | Q | E | séquence 14 |
| E | K | K | Q | A | T | E | E | E | séquence 15 |
| E | E | E | Q | A | T | E | E | E | séquence 16 |
| K | K | S | L | Q | E | R | E | K | séquence 18 |
| E | K | S | R | Q | V | R | E | K | séquence 19 |
| K | K | S | R | Q | V | R | E | K | séquence 20 |

2. Facteur de transcription selon la revendication 1, **caractérisé en ce que** celui-ci est désactivé **par l'ensemble de la délétion de certains sous-domaines du domaine de transactivation du cJun ou par certaines mutations d'endroits de phosphorylation de la séquence d'aminoacide 6 à 194 chez le cJun ou dans la séquence d'aminoacide 1-110 chez ATF2** ou par délétion du domaine à doigt de zinc dans la partie aminoterminale de ATF2 ou la délétion du domaine de fixation dans ATF2 pour des coactivateurs, ou par délétion ou mutation de parties du domaine de fixation à l'ADN.

3. Facteur de transcription selon la revendication 1, **caractérisé en ce que** celui-ci est hyperactivé par substitution de Ser63 et/ou Ser73, Thr89, Thr 91, Thr93 du cJun ou de Thr69 et/ou Thr71, ser90 du ATF2, par Asp, ou par fusion du cJun ou ATF2 avec le domaine de transactivation hyperactif de la protéine du virus herpes simplex VP16.

4. Facteurs de transcription selon l'une quelconque des revendications 1, 2 ou 3 pour l'utilisation comme médicament.

5. Utilisation de facteurs de transcription selon l'une quelconque des revendications 1, 2 ou 3 pour tester des médicaments, dans des cultures cellulaires *in vitro* et des souris transgéniques, dans lesquelles sont exprimés les facteurs de transcription mutants.

6. Utilisation des domaines à fermeture éclair des facteurs de transcription selon l'une quelconque des revendications 1, 2 ou 3 pour coupler différents principes actifs, de préférence des biomolécules.


### SEQUENCE LISTING

<110> FORSCHUNGSZENTRUM KARLSRUHE GMBH
   RJJKS UNIVERSITEIT LEIDEN
   ANGEL, PETER
   VAN DAM, HANS
   VAN DER ERB, ALEX
   HERRLICH, PETER
<120> Transkriptionsfaktoren und deren Verwendung
<130> PLA9822
<140> EP 99 910 361.7
   <141> 1999-03-18
<150> DE 190 15 331.7
   <151> 1998-04-06
<160> 20
<170> Patentln version 3.3
<210> 1
   <211> 31
   <212> PRT
   <213> Unknown
<220>
   <223> Beschreibung der unbekannten Sequenz: Consensus Sequenz
<220>
   <221> misc_feature
   <222> (2)..(7)
   <223> Xaa can be any naturally occurring amino acid
<220>
   <221> misc_feature
   <222> (9)..(14)
   <223> Xaa can be any naturally occurring amino acid
<220>
   <221> misc_feature
   <222> (16)..(21)
   <223> Xaa can be any naturally occurring amino acid
<220>
   <221> misc_feature
   <222> (23)..(28)
   <223> Xaa can be any naturally occurring amino acid
<220>
   <221> misc_feature
   <222> (30)..(31)
   <223> Xaa can be any naturally occurring amino acid
<400> 1
   Leu Xaa Xaa Xaa Xaa Xaa Xaa Leu Xaa Xaa Xaa Xaa Xaa Xaa Leu Xaa
   1 5 10 15
   Xaa Xaa Xaa Xaa Xaa Leu Xaa Xaa Xaa Xaa Xaa Xaa Leu Xaa Xaa
   20 25 30
<210> 2
   <211> 31
   <212> PRT
   <213> Unknown
<220>
   <223> Description of Unkown Sequence: cJun wt
<400> 2
   Leu Glu Glu Lys Val Lys Thr Leu Lys Ala Gln Asn Tyr Glu Leu Ala
   1 5 10 15
   Ser Thr Ala Asn Met Leu Arg Glu Gln Val Ala Gln Leu Lys Gln
   20 25 30
<210> 3
   <211> 31
   <212> PRT
   <213> Unknown
<220>
   <223> Description of Artificial Sequence: Synthetic peptide
<400> 3
   Leu Lys Glu Lys Val Lys Thr Leu Lys Ala Gln Asn Tyr Glu Leu Ala
   1 5 10 15
   Ser Thr Ala Asn Met Leu Arg Glu Gln Val Ala Gln Leu Lys Gln
   20 25 30
<210> 4
   <211> 31
   <212> PRT
   <213> Unknown
<220>
   <223> Description of Artificial Sequence: Synthetic peptide
<400> 4
   Leu Glu Glu Glu Val Lys Thr Leu Glu Ala Gln Asn Tyr Glu Leu Ala
   1 5 10 15
   Ser Thr Ala Asn Met Leu Arg Glu Gln Val Ala Gln Leu Lys Gln
   20 25 30
<210> 5
   <211> 31
   <212> PRT
   <213> Unknown
<220>
   <223> Description of Artificial Sequence: Synthetic
<400> 5
   Leu Glu Glu Lys Val Lys Thr Leu Lys Ala Gln Asn Tyr Glu Leu Ala
   1 5 10 15
   Ser Thr Ala Asn Met Leu Glu Glu Glu Val Ala Gln Leu Lys Gln
   20 25 30
<210> 6
   <211> 31
   <212> PRT
   <213> Unknown
<220>
   <223> Description of Artificial Sequence: Synthetic peptide
<400> 6
   Leu Glu Glu Glu Val Lys Thr Leu Glu Ala Gln Asn Tyr Glu Leu Ala
   1 5 10 15
   Ser Thr Ala Asn Met Leu Glu Glu Glu Val Ala Gln Leu Lys Gln
   20 25 30
<210> 7
   <211> 31
   <212> PRT
   <213> Unknown
<220>
   <223> Description of Artificial Sequence: Synthetic peptide
<400> 7
   Leu Lys Glu Lys Val Lys Thr Leu Glu Ala Gln Asn Tyr Glu Leu Ala
   1 5 10 15
   Ser Thr Ala Asn Met Leu Arg Glu Gln Val Ala Gln Leu Lys Gln
   20 25 30
<210> 8
   <211> 31
   <212> PRT
   <213> Unknown
<220>
   <223> Description of Artificial Sequence: Synthetic peptide
<400> 8
   Leu Lys Glu Glu Val Lys Thr Leu Glu Ala Gln Asn Tyr Glu Leu Ala
   1 5 10 15
   Ser Thr Ala Asn Met Leu Arg Glu Gln Val Ala Gln Leu Lys Gln
   20 25 30
<210> 9
   <211> 31
   <212> PRT
   <213> Unknown
<220>
   <223> Description of Artificial Sequence: Synthetic peptide
<400> 9
   Leu Lys Glu Lys Val Lys Thr Leu Lys Ala Gln Asn Tyr Glu Leu Ala
   1 5 10 15
   Ser Thr Ala Asn Met Leu Glu Glu Glu Val Ala Gln Leu Lys Gln
   20 25 30
<210> 10
   <211> 31
   <212> PRT
   <213> Unknown
<220>
   <223> Description of Artifrcial Sequence: Synthetic peptide
<400> 10
   Leu Lys Glu Glu Val Lys Thr Leu Glu Ala Gln Asn Tyr Glu Leu Ala
   1 5 10 15
   Ser Thr Ala Asn Met Leu Glu Glu Glu Val Ala Gln Leu Lys Gln
   20 25 30
<210> 11
   <211> 31
   <212> PRT
   <213> Unknown
<220>
   <223> Description of Artificial Sequence: Synthetic peptide
<400> 11
   Leu Glu Glu Lys Val Lys Thr Leu Lys Ala Gln Asn Tyr Glu Leu Ala
   1 5 10 15
   Ser Thr Ala Asn Met Leu Arg Glu Gln Val Ala Gln Leu Glu Gln
   20 25 30
<210> 12
   <211> 31
   <212> PRT
   <213> Unknown
<220>
   <223> Description of Artificial Sequence: Synthetic peptide
<400> 12
   Leu Lys Glu Lys Val Lys Thr Leu Lys Ala Gln Asn Tyr Glu Leu Ala
   1 5 10 15
   Ser Thr Ala Asn Met Leu Arg Glu Gln Val Ala Gln Leu Glu Gln
   20 25 30
<210> 13
   <211> 31
   <212> PRT
   <213> Unknown
<220>
   <223> Description of Artificial Sequence: Synthetic peptide
<400> 13
   Leu Glu Glu Lys Val Lys Thr Leu Glu Ala Gln Asn Tyr Glu Leu Ala
   1 5 10 15
   Ser Thr Ala Asn Met Leu Arg Glu Gln Val Ala Gln Leu Glu Gln
   20 25 30
<210> 14
   <211> 31
   <212> PRT
   <213> Unknown
<220>
   <223> Description of Artificial Sequence: Synthetic peptide
<400> 14
   Leu Glu Glu Glu Val Lys Thr Leu Glu Ala Gln Asn Tyr Glu Leu Ala
   1 5 10 15
   Ser Thr Ala Asn Met Leu Arg Glu Gln Val Ala Gln Leu Glu Gln
   20 25 30
<210> 15
   <211> 31
   <212> PRT
   <213> Unknown
<220>
   <223> Description of Artificial Sequence: Synthetic peptide
<400> 15
   Leu Glu Glu Lys Val Lys Thr Leu Lys Ala Gln Asn Tyr Glu Leu Ala
   1 5 10 15
   Ser Thr Ala Asn Met Leu Glu Glu Glu Val Ala Gln Leu Glu Gln
   20 25 30
<210> 16
   <211> 31
   <212> PRT
   <213> Unknown
<220>
   <223> Description of Artificial Sequence: Synthetic peptide
<400> 16
   Leu Glu Glu Glu Val Lys Thr Leu Glu Ala Gln Asn Tyr Glu Leu Ala
   1 5 10 15
   Ser Thr Ala Asn Met Leu Glu Glu Glu Val Ala Gln Leu Glu Gln
   20 25 30
<210> 17
   <211> 31
   <212> PRT
   <213> Unknown
<220>
   <223> Description of Artificial Sequence: ATF2 wt
<400> 17
   Leu Glu Lys Lys Ala Glu Asp Leu Ser Ser Leu Asn Gly Gln Leu Gln
   1 5 10 15
   Ser Glu Val Thr Leu Leu Arg Asn Glu Val Ala Gln Leu Lys Gln
   20 25 30
<210> 18
   <211> 3.1
   <212> PRT
   <213> Unknown
<220>
   <223> Description of Artificial Sequence: Synthetic peptide
<400> 18
   Leu Lys Lys Lys Ala Glu Asp Leu Ser Ser Leu Asn Gly Gln Leu Gln
   1 5 10 15
   Ser Glu Val Thr Leu Leu Arg Asn Glu Val Ala Gln Leu Lys Gln
   20 25 30
<210> 19
   <211> 31
   <212> PRT
   <213> Unknown
<220>
   <223> Description of Artificial Sequence: Synthetic peptide
<400> 19
   Leu Glu Lys Lys Ala Glu Asp Leu Ser Ser Arg Asn Gly Gln Leu Gln
   1 5 10 15
   Ser Val Val Thr Leu Leu Arg Asn Glu Val Ala Gln Leu Lys Gln
   20 25 30
<210> 20
   <211> 31
   <212> PRT
   <213> Unknown
<220>
   <223> Description of Artificial Sequence: Synthetic peptide
<400> 20
   Leu Lys Lys Lys Ala Glu Asp Leu Ser Ser Leu Asn Gly Gln Leu Gln
   1 5 10 15
   Ser Val Val Thr Leu Leu Arg Asn Glu Val Ala Gln Leu Lys Gln
   20 25 30